# EUROPÄISCHE PATENTSCHRIFT

(11) **EP 1 604 624 B1**
(45) Veröffentlichungstag und Bekanntmachung des Hinweises auf die Patenterteilung: **23.01.2008**
(21) Anmeldenummer: 05012076.5
(22) Anmeldetag: 04.06.2005
(51) Int. Cl.: A61F 5/02

(54) **Korsett, insbesondere zur Skoliosebehandlung**
Corset, especially for the treatment of scoliosis
Corset, en particulier pour le traitement de la scoliose

(30) Priorität: 11.06.2004 DE 102004028505
(43) Veröffentlichungstag der Anmeldung: 14.12.2005
(73) Patentinhaber: Weiss, Grita, 55566 Bad Sobernheim (DE)
(72) Erfinder: Weiss, Grita, 55566 Bad Sobernheim (DE)
(74) Vertreter: Becker, Bernd

(56) Entgegenhaltungen:
- DE-A1- 3 622 265
- DE-A1- 4 014 338
- DE-C- 931 975
- DE-U1- 20 204 936
- US-A- 3 274 996
- US-A- 6 010 472
- US-A1- 2004 167 449

## Beschreibung

Die Erfindung bezieht sich auf ein Korsett, insbesondere zur Skoliosebehandlung, umfassend vordere und hintere flächige Druckkörper, wobei Ausweichraum für verdrängtes Rumpfvolumen vorgesehen ist.

Die US-A-6 010 472 offenbart eine Hyperextensions-Orthese in Rahmenbauweise mit mehreren am Körper eines Patienten aufliegenden Pelotten, wobei zwei kurz unterhalb der beiden Schlüsselbeine aufliegende und mit der übrigen Orthese starr verbundene Pelotten vorhanden sind.

Zur Behandlung von Wirbelsäulenverbiegungen, insbesondere Skoliose, sind verschiedene Korsettypen entwickelt worden. Diese umfassen unter anderen die so genannten teilaktiven starren Korsette, beispielsweise das Cheneau-, CBW-, Boston-, Stagnara-, Milwaukee-Korsett, und haben einerseits Druckzonen und andererseits Freiräume. Auf vorstehende Bereiche, wie Rippenbuckel, wird Druck ausgeübt und gegenüberliegend ist Freiraum vorgesehen, damit die fortgedrückten Bereiche dorthin ausweichen. Die meisten vorhandenen Rumpforthesen wirken über seitliche Kräfte und wirken dabei mehr oder weniger auch auf den Rippenbuckel und/oder den Lendenwulst eine Derotationskraft (Entdrehkraft) aus. Lediglich beispielhaft werden aufgeführt ein Korsett nach der DE 29 21 015 C2 zur aktiven Korrektur der Wirbelsäule mit fortschreitender Regulierung und eine Variante eines Cheneau-Korsetts nach der DE 36 22 265 A1 mit abnehmbarem Halsteil, das zur Therapie von hochthorakalen und cervikalen Verkrümmungen und bei Überhang dient.

Wichtig ist indessen die Behandlung nicht nur der Seitverbiegung, sondern auch die Berücksichtigung der sagittalen Verbiegung, was erst in neuerer Zeit berücksichtigt wird. Die nicht korrigierten Wirbelsäulenverbiegungen können langfristig zu Beeinträchtigungen der Funktion und zu Schmerzen führen. Da vielfach eine fast ganztägige Tragezeit des Korsetts über etliche Jahre benötigt wird, um langfristig eine Korrektur zu erzielen, sollte ein Korsett möglichst bequem zu tragen sein. Weiter mitbestimmend für die Akzeptanz des Korsetts und damit auch für den Therapieerfolg ist dessen Unauffälligkeit beim Tragen.

Mit dem so genannten Rigo-System und dem Cheneau-Korsett soll zusätzlich zur Korrektur der Seitverbiegung und Verdrehung der Wirbelsäule, insbesondere im Brustwirbelsäulenbereich, die Rückrundung der Brustwirbelsäule wieder hergestellt werden.

Der Erfindung liegt die Aufgabe zugrunde, ein Korsett zur Behandlung von Wirbelsäulendeformitäten, insbesondere zur Skoliosebehandlung, zu schaffen, das eine wirksame Korrektur erzielt und zugleich günstige Trageeigenschaften aufweist.

Diese Aufgabe ist erfindungsgemäß bei einem Korsett mit den Merkmalen des Anspruchs 1 gelöst. Vorteilhafte Weiterbildungen des erfindungsgemäßen Korsetts sind Gegenstand der Unteransprüche.

Ein erfindungsgemäßes Korsett, insbesondere zur Skoliosebehandlung, umfasst somit vordere und hintere flächige Druckkörper, wobei Ausweichraum für verdrängtes Rumpfvolumen vorgesehen ist. Ein unterer vorderer flächiger Druckkörper ist im mittigen Bereich vorgesehen. Weiter ist ein oberer vorderer flächiger Druckkörper vorgesehen, der eine etwa horizontale Erstreckung aufweist und sich seitlich zur Mitte hin erstreckt. Dabei sind der untere und der obere flächige Druckkörper durch mindestens einen Längsträger verbunden. Außerdem ist ein mittlerer hinterer flächiger Druckkörper vorgesehen. Die Druckkörper sind so positioniert, dass der untere vordere Druckkörper direkt oberhalb des Schambeins beidseits auf den untersten Teil der Abdominalfaszie drückt, der obere vordere Druckkörper kurz oberhalb des Rippenbogens auf die Rippen drückt und der mittlere hintere Druckkörper auf die mittlere Lendensäule drückt.

Grundprinzip der Wirkungsweise des erfindungsgemäßen Korsetts ist, dass es zwei vordere, nach hinten gerichtete Druckzonen und eine diesen gegenüberstehende hintere, nach vorne gerichtete Druckzone hat. Etwas oberhalb und leicht seitlich vom Schambein beidseits befindet sich die erste Druckzone (gebildet durch die unteren vorderen flächigen Druckkörper), die auf den untersten Teil der Abdominalfaszie, d.h. den unteren Anteil der Bindegewebsplatte des Bauches drückt, und durch die das Becken nach hinten korrigiert wird. In Bezug auf diese erste Druckzone wirkt die zweite vordere Druckzone (mittels der oberen vorderen Druckkörper), die etwa drei querfingerbreit oberhalb des Rippenbogens von vorne einwirkt und die Rippen der mittleren Brustwirbelsäule nach hinten schieben soll. Damit soll eine Korrektur der zu flachen Brustwirbelsäule nach hinten in die Rundwölbung erfolgen. Der bei einer Brustkorbskoliose vorne vorstehende Rippenbuckel wird nach hinten korrigiert und entdreht. Die Korrektur der Lendenwirbelsäule und der Brustwirbelsäule erfolgt widerlagernd. Die dritte Druckzone, eine symmetrische Lendendruckzone (in Form des mittleren hinteren flächigen Druckkörpers), steht den beiden anderen Druckzonen als Widerlager gegenüber. Sie ist der mittleren Lendenwirbelsäule angepasst und korrigiert die zu flache Lendenwirbelsäule nach vorne, indem sie die Hohlwölbung der Lendenwirbelsäule verstärkt. Ein eventuell bestehender Lendenwulst wird so automatisch mehr als das Lendental redressiert und korrigiert.

Als Freiraum für die Druckrichtung von hinten nach vorne im Lendenwirbelsäulenbereich ist der Bauch um den Bauchnabel herum freizuhalten, d.h. hier müssen Expansionsräume in geeigneter Weise eingerichtet werden, um das durch die dritte Druckzone nach vorne verschobene Rumpfvolumen aufzufangen. Daher sind bevorzugt der untere und der obere Druckkörper durch zwei schräg verlaufende Längsträger verbunden, die sich von den seitlichen Außenbereichen des oberen vorderen Druckkörpers zu dem unteren vorderen Druckkörper erstrecken und nach vorne gebogen sind, und zwischen dem unteren und dem oberen Druckkörper und den beiden Längsträgern ist ein vorderer Ausweichraum vorgesehen. Dies ist auch der Grund für die Vorbiegung der stabilen Längsträger, die die beiden vorderen Druckzonen verbinden.

Durch die erfindungsgemäße Korsettbauweise besteht eine au-ßerordentlich große Bewegungsmöglichkeit für den Korsettträger. Das Korsettgewicht ist herabgesetzt und insbesondere ist das Korsett unter der Kleidung so gut wie nicht zu sehen.

Eine seitliche Anordnung des Verschlusses zwischen dem mittleren hinteren Druckkörper und den vorderen Druckkörpern ermöglicht eine besonders gute Redressierung und Korrektur des Lendenwulstes und wird bei eher lumbalen Krümmungen gewählt. Wird der Verschluss dort zugezogen (langer Hebel), so ergibt sich auf der gegenüberliegenden Seite im Bereich des Lendenwulstes (kurzer Hebel) aufgrund der Biegungsstabilität des hinteren Druckkörpers eine große lokale Kraft. Diese Kraft bewirkt die Redressierung bzw. Korrektur des Lendenwulstes. Ihre Wirkung wird als "Nussknacker-Effekt" bezeichnet.

Das erfindungsgemäße Korsett eignet sich dazu, auch teileingesteifte Wirbelsäulendeformitäten zu redressieren, da durch die Verwendung der flächenförmigen Druckkörper eine ausreichende Redressionskraft bzw. Korrekturkraft aufgebracht werden kann. Dies ist vor allem durch die sagittale Widerlagerung mit günstigen Hebel-/Kraftarm-Verhältnissen ermöglicht, die statt herkömmlich 0,5 zu 1 nunmehr 2 zu 4 für die Kraftverhältnisse bei der Einwirkung auf den Brustkorb von vorne ergeben.

Mit dem erfindungsgemäßen Korsett werden kurzfristig sogar bessere Korrekturergebnisse als bei einem herkömmlichen Korsett erzielt. Dabei sind die Korrektureffekte für die Seitverbiegung wie auch für die Buckelkorrektur bei Überkorrektur des seitlichen Wirbelsäulenprofils dieselben. Wichtig ist, dass die Hohlwölbung der Lendenwirbelsäule in die Extremform gebracht wird (Überkorrektur) und die Rundwölbung der Brustwirbelsäule mittels Widerlager nach hinten forciert wird.

Das erfindungsgemäße Korsett eignet sich insbesondere zur Behandlung von idiopathischer Skoliose, jedoch auch für alle anderen Skoliosen mit ähnlich gestörtem Sagittalprofil. Des Weiteren findet es Einsatz zur Behandlung von Kreuzschmerzen bei Reduktion der Lendenlordose oder Kyphose im Lendenbereich und zur Behandlung der schmerzhaften Skoliose im späteren Erwachsenenalter mit dieser entsprechender Deformität. Es kann auch bei Morbus Scheuermann im Lendenbereich angewendet werden. Nicht eingesetzt werden kann das erfindungsgemäße Korsett bei allen Deformitäten mit Verstärkung der Wirbelsäulenrundwölbungen im Brustwirbelsäulenbereich nach hinten (Kyphose) oder einer Verstärkung der Hohlwölbung im Lendenbereich (Lordose), somit beim thorakalen Morbus Scheuermann, thorakolumbalen Morbus Scheuermann und Spondylolisthesis (Wirbelgleiten) im unteren Lendenwirbelbereich.

Insbesondere zur Behandlung von thorakalen Krümmungen, bei der die Rundwölbung im Brustwirbelsäulenbereich wiederhergestellt werden soll, sind vorzugsweise die vorderen Druckkörper geteilt. Dort befindet sich dann auch der Korsettverschluss, der zweckmäßig verstellbar ist. Durch Zusammenziehen der oberen Verschlussschnalle wird die obere Druckzone verstärkt und damit die nach hinten wirkende Kraft vergrößert.

Vorteilhaft sind die Pelottenträger leicht nach vorne vorgebogen, so dass die Kraftentfaltung der vorderen Druckzone über den vorderen Verschluss ohne die Gefahr der Gewebeverletzung oder Hauteinklemmung möglich wird.

Eine mittige Teilung der vorderen Druckkörper und Verbindung mit Verstellmöglichkeit ermöglicht eine Breitenverstellung des Korsetts. Bei dieser Anordnung ist die Stabilität des Korsetts nicht beeinträchtigt. Eine mittige Teilung des hinteren Druckkörpers kann auch zum Einbau einer Verstellmöglichkeit genutzt werden.

Wird der Verschluss des Korsetts seitlich vorgesehen, muss ebenfalls eine Anpass-, d.h. Verstellmöglichkeit vorgesehen werden. Diese sollte dem Verschluss gegenüberliegend sein. Es kann auch ein Gelenk vorgesehen werden, um einen langen Verschlusshebel zu schaffen und damit den der erfindungsgemäßen Konstruktion eigenen "Nussknacker-Effekt" herbeizuführen.

Durch eine solche Anordnung der Verschlüsse kann der bei Wirbelsäulendeformitäten, insbesondere bei größeren Skoliosen vorliegender Einsteifung, benötigten größeren Anforderung an die Korsettstabilität Rechnung getragen werden.

Eine besonders zweckmäßige Ausgestaltung des erfindungsgemäßen Korsetts ergibt sich, wenn der obere flächige Druckkörper die Form eines etwa quer verlaufenden Bügels hat. Dadurch kann Material eingespart werden und der Druckkörper kann, insbesondere bei Teilung, in Art einer Spange wirken.

Ebenso sind die beiden Längsträger bevorzugt in ihrem unteren Ende zu dem unteren flächigen Druckkörper verbreitert, wodurch sich eine integrierte Ausführung ergibt. Zweckmäßig geht auch der obere flächige Druckkörper in den seitlichen Außenbereichen jeweils in die Längsträger über und der obere flächige Druckkörper am seitlichen Ende nach hinten und unten in einen gewölbten Bereich über. Auch können die vorderen flächigen Druckkörper und/oder der hintere flächige Druckkörper einstückig ausgebildet sein, wodurch die Eigensteifigkeit des Korsettgerüsts hoch ist.

Obwohl das Korsett aus Einzelteilen aufgebaut sein kann, ergibt sich ein kompakter und stabiler Aufbau. Dies ist insbesondere die Folge dessen, dass der mittlere hintere Druckkörper mit den vorderen Druckkörpern, vorzugsweise im gewölbten Bereich, verbunden ist. Je nach Anpassung und Form der Druckkörper und der Verbindungsfixierung ergibt sich so ein außerordentliches stabiles und steifes Korsett mit wirksamen Korrekturkräften. Infolge der durch die freie Bauweise ermöglichten Freiräume ist der Tragekomfort für den Patienten indessen erheblich.

Das erfindungsgemäße Korsett kann auf verschiedene Weise ausgebildet sein. Es kann als Rahmenkonstruktion mit stabilen Metallträgern, vorgeformten Kunststoffpelotten, Polstermaterial, Verschlüssen, Verstellmechanismen aufgebaut sein. Alternativ kann es als Gusskonstruktion aus Kunststoff ausgebildet sein, wobei statt der Metallträger der Rahmenkonstruktion gegossene Kunststoffflächenelemente bzw. -platten oder - bügel zur Aussteifung dienen. Die Bauteile des Korsetts können auch durch Tiefziehen auf einem Gips- oder Kunststoffschaumpositiv hergestellt werden, wobei zur Versteifung der Bauteile diese mit Profilierung ausgestattet werden.

Die Erfindung wird im Folgenden weiter anhand von Ausführungsbeispielen und der Zeichnung beschrieben. Diese Darstellung dient lediglich zur Veranschaulichungszwecken und soll die Erfindung nicht auf die konkret angegebenen Merkmalskombinationen einschränken. Es zeigen:
- Fig. 1a bis Fig. 1d: eine Vorder-, Rück- und zwei Seitenansichten eines ersten Ausführungsbeispiels eines erfindungsgemäßen Korsetts,
- Fig. 1e bis Fig. 1g: die sagittale Widerlagerung und Kräfteangriffspunkte bei dem erfindungsgemäßen Korsett und im Vergleich dazu bei einem herkömmlichen Rahmenkorsett, um die Hebelarmverhältnisse zu veranschaulichen,
- Fig. 1h und Fig. 1i: anhand einer Skelettdarstellung die Kräfteangriffspunkte beim erfindungsgemäßen Korsett und die sich ergebenden Korrekturen,
- Fig. 2a bis Fig. 2e: eine Vorder-, Rück-, zwei Seitenansichten und eine vergrößerte Rückenansicht von oben eines erfindungsgemäßen Korsetts gemäß einem zweiten Ausführungsbeispiel,
- Fig. 3a bis Fig. 3d: eine perspektivische Vorderansicht, eine Rückansicht und zwei perspektivische Vorderansichten eines erfindungsgemäßen Korsetts gemäß einem dritten Ausführungsbeispiel und
- Fig. 4a und Fig. 4b: zwei Vorderansichten eines erfindungsgemäßen Korsetts gemäß einem vierten Ausführungsbeispiel im geschlossenen und im geöffneten Zustand des oberen vorderen Verschlusses.

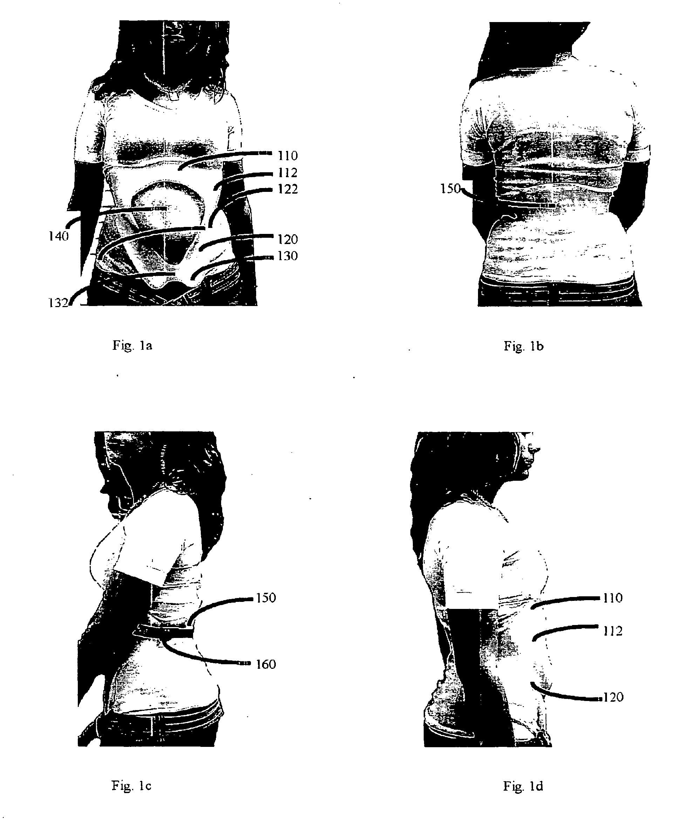

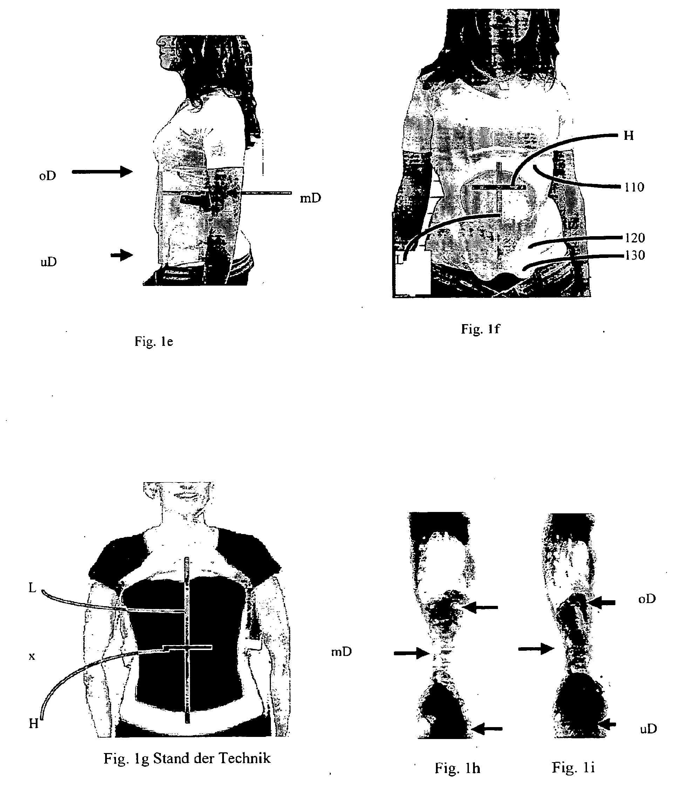

Als erstes wird der Aufbau des erfindungsgemäßen Korsetts anhand von Fig. 1 erläutert. Das Korsett hat drei Druckzonen, von denen zwei von vorne und eine von hinten auf die Wirbelsäule einwirkt. Die Druckzonen sind ausgebildet als flächige Druckkörper, wobei das dargestellte Korsett aus Kunststoff in Form einer Vorderschale und eines Rückenbügels aus Kunststoff geformt ist. Die Vorderschale umfasst den Bauchraum und umgreift diesen seitlich. In Rippenhöhe ist ein oberer Druckkörper 110 ausgebildet, der sich etwa horizontal oberhalb des Rippenbogens erstreckt und vorgesehen ist, um einen Druck nach hinten auszuüben. Der obere vordere Druckkörper 110 erstreckt sich von der Seite bis zur Mitte durchgehend, wobei in der Mitte des so gebildeten bügelartigen Körpers etwas weniger und mehr Druck über die Rippen nach hinten ausgeübt wird. Am seitlichen Ende geht der obere flächige Druckkörper 110 nach hinten und unten in einen gewölbten Bereich 112 über. Von den seitlichen Außenbereichen des oberen flächigen Druckkörpers 110 ist ein Übergang zu Längsträgern 120 gebildet, die die Gestalt breiter Leisten mit konvexer Einwölbung 122 haben und sich zur Körpermitte hin nach unten bis zum untersten Teil der Abdominalfaszie hin erstrecken, wo sie mit ihrem Ende in einen unteren vorderen flächigen Druckkörper 130 übergehen, der direkt oberhalb des Schambeines beiderseits auf den untersten Teil der Abdominalfaszie, d.h. den unteren Anteil der Bindegewebsplatte des Bauches, drückt. Ganz in der Mitte ist eine entlastende Vorwölbung 132 des unteren flächigen Druckkörpers 130 vorgesehen. Der untere flächige Druckkörper 130 befindet sich im mittigen Bereich und stellt eine ebenfalls nach hinten gerichtete zweite Druckzone dar. Die Gestalt der vorderen Korsettschale ist somit insgesamt, von vorne gesehen, in Art eines Dreieckbügels, da in der Mitte der Schale weiträumig ein Ausweichraum 140 für verdrängtes Rumpfvolumen ausgespart ist. Von der Seite gesehen bildet die vordere Korsettschale ebenfalls eine annähernde Dreieckform, wobei jedoch hier die Spitze des Dreiecks in der Körperseite und die Grundlinie entsprechend der Körpervorderseite angeordnet ist. Innen zum Ausweichraum 140 hin sind die Randbereiche der vorderen Korsettschale in Richtung Körpervorderseite leicht nach außen gewölbt, so dass Druckstellen weitgehend vermieden werden.

Die vordere Korsettschale ist mit einem rückwärtigen bügelartigen flächigen Druckkörper 150 verbunden, der sich höhenmäßig zwischen dem oberen und unteren Druckkörper jeweils befindet. Dieser dritte Druckkörper 150 übt eine nach vorne gerichtete Kraft auf die mittlere Lendenwirbelsäule aus. In der Körpermitte ist dieser Bügelkörper verbreitert, um das Auftreten von Druckstellen zu vermindern, wobei außerdem wiederum die Randbereiche etwas vom Körper fort gebogen sind. Der hintere Druckkörper 150 und die vordere Korsettschale (oberer Drehkörper 110, Längsträger 120, unterer Druckkörper 130) sind miteinander verbunden, wobei eine feste Verbindung auf der linken Körperseite (in Fig. 1a rechts) und ein verstellbarer Verschluss 160 auf der rechten Körperseite (in Fig. 1a links) vorgesehen ist, der in Fig. 1c zu sehen ist.

Fig. 1e und 1f zeigen den Ort der Krafteinwirkung, veranschaulicht mit Teildarstellung der das Korsett tragenden Person. Die Vorderansicht von Fig. 1f zeigt eine senkrechte Mittenlinie L und eine quer verlaufende Horizontallinie H, die in Höhe des Kraftangriffspunkts des hinteren flächigen Druckkörpers 150 liegt. Die Höhe des Schnittpunkts ist neben Fig. 1f mit mD veranschaulicht. Die Höhe der nach hinten gerichteten unteren und oberen Druckkraft ist mit uD und mit oD wiederum links von Fig. 1f dargestellt. Damit ist das Prinzip des Korsetts verdeutlicht. Durch die abwechselnde Anordnung von nach vorne und nach hinten gerichteten Kräften ergeben sich Kraftdrehmomente und Hebelkräfte auch in Folge der Widerlagerkonstruktion, wobei die Hebelverhältnisse stark verschieden sind und sich ein kurzer (oD, mD) und ein langer (mD, uD) Hebelarm ergeben. Diese sagittale Widerlagerung mit relativ stark wirkenden Kräften wird auch als "Nussknacker-Prinzip" beschrieben.

Fig. 1g veranschaulicht demgegenüber die wirksamen Kräfte bei einem herkömmlichen Rahmenkorsett. Hier ergeben sich keine Kräfteverhältnisse von 2 bis 4, sondern bestenfalls von 0,5 bis 1, wie auch die Lage des Schwenkpunkts, dargestellt durch das Kreuz links von Fig. 1, veranschaulicht. Damit ist der Korrektureffekt weitaus geringer als bei dem erfindungsgemäßen Korsett.

Die physiologische Einwirkung auf das Skelett und die durch diese Druckkräfte erfolgte Korrektur veranschaulichen Fig. 1h und 1i. Durch die Druckzone uD wird das Becken nach hinten, durch die mittlere Druckzone mD die zu flache Lendenwirbelsäule nach vorne und durch die obere Druckzone oD die zu flache Brustwirbelsäule wiederum nach hinten korrigiert. Dabei erfolgt die Korrektur von Lendenwirbelsäule und Brustwirbelsäule im Widerlager.

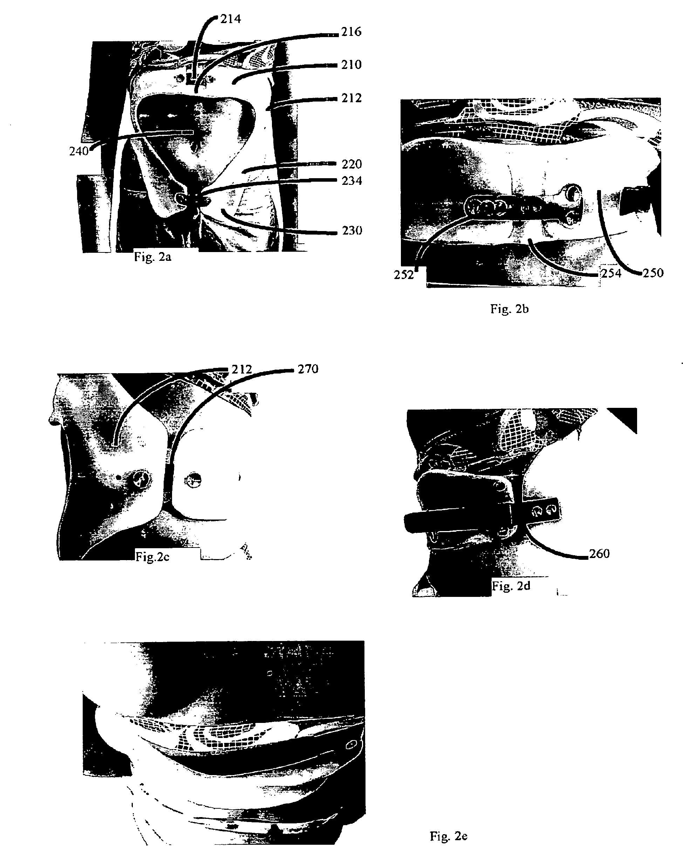

Fig. 2 veranschaulicht ein zweites Ausführungsbeispiel des Korsetts, diesmal mit geteilten Druckkörpern 210, 230, 250. Die Gestalt des Korsetts ist grundsätzlich ähnlich dem zuvor beschriebenen, wobei jedoch seine Form an die durchzuführende Korrektur und Körpergestalt des Trägers angepasst ist. Der obere vordere flächige Druckkörper 210 geht nach hinten und unten in den gewölbten Bereich 212 über und ist unter Ausbildung des Ausweichraums 240 über die Längsträger 220 mit dem unteren vorderen flächigen Druckkörper 230 verbunden. Im Weiteren sind der obere vordere flächige Druckkörper 210 und der untere vordere flächige Druckkörper 230 geteilt und durch verstellbare Verbindungsstücke 214, 234 verbunden, wobei unterhalb von diesen jeweils Polstermaterial 216 vorgesehen ist, um das Auftreten von Druckstellen zu vermeiden. Der mittlere hintere Druckkörper 250 ist ebenfalls geteilt und durch einen verstellbaren Verbindungsbügel 252 sind die beiden Hälften fest miteinander verbunden. Zur Verhinderung von Druckstellen ist Polstermaterial 254 vorgesehen. Ebenso ist der mittlere hintere Druckkörper 250 außerhalb der Druckzonen vom Körper fort gebogen, wie die Ansicht von Fig. 2d für den oberen Rand deutlich zeigt. Der bügelartige hintere Druckkörper 250 ist mit der vorderen Korsettschale einerseits durch ein Verbindungsstück 270 und andererseits durch einen verstellbaren schnallenartigen Verschluss 260 verbunden. Wird gemäß Rückenansicht von Fig. 2e der rechts befindliche Verschluss zugezogen (langer Hebel), entsteht links im Bereich des Lendenwulstes (kurzer Hebel) der beschriebene "Nussknacker-Effekt".

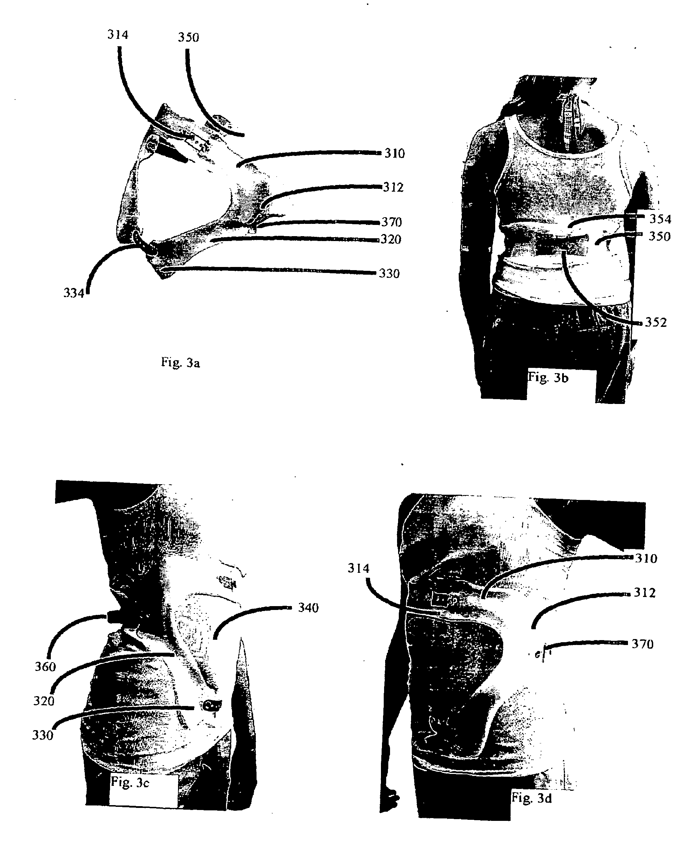

Ein drittes Ausführungsbeispiel des Korsetts ist in den Fig. 3a bis 3d dargestellt, bei dem, wie bereits erläutert, der obere vordere flächige Druckkörper 310 nach hinten und unten in den gewölbten Bereich 312 übergeht und unter Ausbildung des Ausweichraums 340 über die Längsträger 320 mit dem unteren vorderen flächigen Druckkörper 330 verbunden ist. Deutlich ist seine Konstruktion anhand der perspektivischen Ansicht von Fig. 3a zu sehen. Wiederum ist die vordere Korsettschale hälftig geteilt, d.h. die beiden vorderen Druckkörper 310, 330 sind geteilt und die beiden Teile des jeweiligen Druckkörpers 310, 330 durch verstellbare Verbindungsstücke 314, 334 miteinander verbunden. Ebenso ist der mittlere hintere Druckkörper 350 geteilt und durch den verstellbaren Verbindungsbügel 352 sind dessen beiden Hälften fest miteinander verbunden, wobei im Bereich des Verbindungsbügels 352 Polstermaterial 354 vorgesehen ist. Der bügelartige hintere Druckkörper 350 ist mit der vorderen Korsettschale einerseits durch das Verbindungsstück 370 und andererseits durch den verstellbaren schnallenartigen Verschluss 360 verbunden. Von der Konstruktion her entspricht dieses Korsett im Wesentlichen dem vorgehend beschriebenen, so dass auch auf Grund einer entsprechenden Bezeichnung der Nummerierung der Bezugszeichen die Analogie deutlich erkennbar ist.

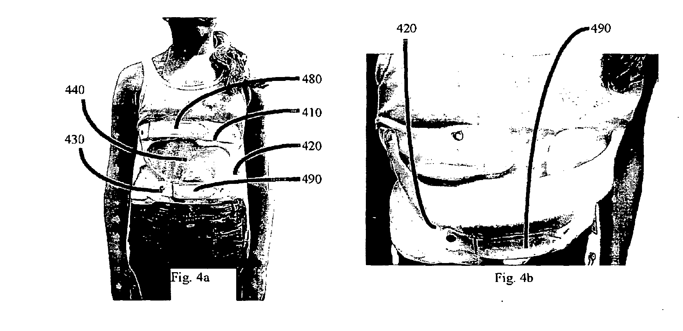

Ein viertes Ausführungsbeispiel des Korsetts ist in den Fig. 4a und 4b anhand von zwei Vorderansichten veranschaulicht. Die Konstruktion des Vorderteils des Korsetts ähnelt grundsätzlich den bisher beschriebenen Ausführungsbeispielen, wobei der obere vordere Druckkörper 410 unter Ausbildung des Ausweichraums 440 über die Längsträger 420 mit dem unteren vorderen flächigen Druckkörper 430 verbunden ist. Indessen sind Verschlüsse 480, 490 in diesem Fall an der Vorderseite des Korsetts vorgesehen, und zwar in Form einer oberen und einer unteren Verschlussschnalle. Durch das Zusammenziehen der oberen Verschlussschnalle wird die obere Druckzone verstärkt und mehr Kraft nach hinten ausgeübt. Dieses Korsett dient zur Korrektur einer thorakalen Krümmung, d.h. die Rundwölbung soll im Brustwirbelsäulenbereich wieder hergestellt werden.

## Patentansprüche

1. Korsett, insbesondere zur Skoliosebehandlung, umfassend vordere und hintere flächige Druckkörper, wobei Ausweichraum (140, 240, 340, 440) für verdrängtes Rumpfvolumen vorgesehen ist, **dadurch gekennzeichnet, dass**
- ein unterer vorderer flächiger Druckkörper (130, 230, 330, 430) im mittigen Bereich vorgesehen ist,
- ein oberer vorderer flächiger Druckkörper (110, 210, 310, 410) vorgesehen ist, der eine etwa horizontale Erstreckung von der einen Seite zur Mitte hin bis zur anderen Seite aufweist,
- wobei der untere und der obere flächige Druckkörper (110, 130; 210, 230; 310, 330; 410) durch mindestens einen Längsträger (120, 220, 320, 420) verbunden sind,
- und ein mittlerer hinterer flächiger Druckkörper (150, 250, 350) vorgesehen ist,
- wobei die Druckkörper so positioniert sind, dass beim Gebrauch des Korsetts der untere vordere Druckkörper (130, 230, 330, 430) direkt oberhalb des Schambeins beidseits auf den untersten Teil der Abdominalfaszie drückt, der obere vordere Druckkörper (110, 210, 310, 410) drei querfingerbreit oberhalb des Rippenbogens auf die Rippen drückt und der mittlere hintere Druckkörper (150, 250, 350) auf die mittlere Lendensäule drückt.

2. Korsett nach Anspruch 1, **dadurch gekennzeichnet, dass** der untere und der obere Druckkörper (110, 130; 210, 230; 310, 330; 410) durch zwei schräg verlaufende Längsträger (120, 220, 320, 420) verbunden sind, die sich von den seitlichen Außenbereichen des oberen vorderen Druckkörpers (110, 210, 310, 410) zu dem unteren vorderen Druckkörper (130, 230, 330, 430) erstrecken und nach vorne gebogen sind, und zwischen dem unteren und dem oberen Druckkörper (110, 130; 210, 230; 310, 330; 410) und den beiden Längsträgern (120, 220, 320, 420) ein vorderer Ausweichraum (140, 240, 340, 440) vorgesehen ist.

3. Korsett nach Anspruch 1, **dadurch gekennzeichnet, dass** der obere vordere Druckkörper (210, 310, 410) geteilt ist.

4. Korsett nach Anspruch 1 oder 3, **dadurch gekennzeichnet, dass** der untere vordere Druckkörper (230, 330, 430) geteilt ist.

5. Korsett nach einem der Ansprüche 1 bis 4, **dadurch gekennzeichnet, dass** der mittlere hintere Druckkörper (250, 350) geteilt ist.

6. Korsett nach einem der Ansprüche 1 bis 5, **dadurch gekennzeichnet, dass** der obere flächige Druckkörper (110, 210, 310, 410) die Form eines etwa quer verlaufenden Bügels hat.

7. Korsett nach einem der Ansprüche 1 bis 6, **dadurch gekennzeichnet, dass** die beiden Längsträger in ihrem unteren Ende zu dem unteren flächigen Druckkörper verbreitert sind.

8. Korsett nach einem der Ansprüche 1 bis 7, **dadurch gekennzeichnet, dass** der obere flächige Druckkörper (110, 210, 310, 410) in den seitlichen Außenbereichen jeweils in die Längsträger (120, 220, 320, 420) übergeht.

9. Korsett nach einem der Ansprüche 1 bis 8, **dadurch gekennzeichnet, dass** der obere flächige Druckkörper (110, 210, 310) am seitlichen Ende nach hinten und unten in einen gewölbten Bereich (112, 212, 312) übergeht.

10. Korsett nach einem der Ansprüche 1 bis 9, **dadurch gekennzeichnet, dass** der mittlere hintere Druckkörper (150, 250, 350) mit den vorderen Druckkörpern (110, 130; 210, 230; 310, 330) verbunden ist.

11. Korsett nach Anspruch 9, **dadurch gekennzeichnet, dass** der mittlere hintere Druckkörper (150, 250, 350) mit dem gewölbten Bereich (112, 212, 312) verbunden ist.

12. Korsett nach einem der Ansprüche 1 bis 11, **dadurch gekennzeichnet, dass** der gewölbte Bereich (112, 212, 312) auf der Höhe der voreren Achselhöhlenlängslinie derart ausgebildet ist, dass ein Expansionsraum für das nach vorne redressierte Rumpfvolumen bereitgestellt wird.

13. Korsett nach Anspruch 12, **dadurch gekennzeichnet, dass** der gewölbte Bereich (112, 212, 312) im Bereich des Expansionsraums Versteifungsmittel aufweist.

14. Korsett nach einem der Ansprüche 1 bis 13, **dadurch gekennzeichnet, dass** der Verschluss (160, 260, 360) seitlich zwischen dem mittleren hinteren Druckkörper und den vorderen Druckkörpern vorgesehen ist.

15. Korsett nach einem der Ansprüche 3 und 4 bis 11, **dadurch gekennzeichnet, dass** der Verschluss (480, 490) vorne vorgesehen ist.

16. Korsett nach Anspruch 15, **dadurch gekennzeichnet, dass** die Längsträger (120, 220, 320, 420) leicht nach vorne vorgebogen sind.

17. Korsett nach einem der Ansprüche 1 bis 16, **dadurch gekennzeichnet, dass** die Anordnung und Druckkraft der flächigen Druckkörper verstellbar ist.

18. Korsett nach den Ansprüchen 3 bis 5 und 16, **dadurch gekennzeichnet, dass** die geteilten vorderen und/oder hinteren flächigen Druckkörper (214, 234, 252; 314, 334, 352) jeweils durch einen Verstellmechanismus verbunden sind.

19. Korsett nach einem der Ansprüche 1 bis 18, **dadurch gekennzeichnet, dass** zwischen den vorderen und dem hinteren flächigen Druckkörper ein Verstellmechanismus (170, 270, 370) vorgesehen ist.

20. Korsett nach einem der Ansprüche 1 bis 18, **dadurch gekennzeichnet, dass** die vorderen flächigen Druckkörper (110, 130) einstückig ausgebildet sind.

21. Korsett nach einem der Ansprüche 1 bis 20, **dadurch gekennzeichnet, dass** der hintere flächige Druckkörper (150) einstückig ausgebildet ist.

22. Korsett nach einem der Ansprüche 1 bis 21, **dadurch gekennzeichnet, dass** es als Rahmenkonstruktion aufgebaut ist.

23. Korsett nach einem der Ansprüche 1 bis 21, **dadurch gekennzeichnet, dass** es als Gusskonstruktion aus Kunststoff ausgebildet ist.

24. Korsett nach einem der Ansprüche 1 bis 21, **dadurch gekennzeichnet, dass** es durch Tiefziehen mit Profilierung aus Kunststoff ausgebildet ist.

## Claims

1. Corset, more especially for the treatment of scoliosis, the said corset including front and rear flat pressure bodies, wherein yielding space (140, 240, 340, 440) is provided for displaced trunk volume,
**characterised in that**
- a lower front flat pressure body (130, 230, 330, 440) is provided in the central region ,
- an upper front flat pressure body (110, 210, 310, 410) is provided and includes a substantially horizontal extension from the one side towards the centre and on to the other side,
- wherein the lower and the upper flat pressure bodies (110, 130; 210, 230; 310, 330; 410) are connected by at least one longitudinal support (120, 220, 320, 420),
- and a central rear flat pressure body (150, 250, 350) is provided,
- wherein the pressure bodies are positioned such that when the corset is used, the lower front pressure body (130, 230 330, 430) presses directly above the pubic bone on both sides on the lowest part of the abdominal facia, the upper front pressure body (110, 210, 310, 410) presses the width across three fingers above the costal arch onto the ribs and the central rear pressure body (150, 250, 350) presses onto the central lumbar column.

2. Corset according to claim 1 **characterised in that** the lower and the upper pressure bodies (110, 130; 210, 230; 310, 330; 410) are connected by two inclinedly extending longitudinal supports (120, 220, 320, 420), which extend from the lateral outside regions of the upper front pressure body (110, 210, 310, 410) to the lower front pressure body (130, 230, 330, 430) and are curved forwards, and between the lower and the upper pressure body (110, 130; 210, 230, 310, 330; 410) and the two longitudinal supports (120, 220, 320, 420) there is provided a front yielding space (140, 240, 340, 440).

3. Corset according to claim 1, **characterised in that** the upper front pressure body (210, 310, 410) is divided.

4. Corset according to claim 1 or 3, **characterised in that** the lower front pressure body (230, 330, 430) is divided.

5. Corset according to one of claims 1 to 4, **characterised in that** the central rear pressure body (250, 350) is divided.

6. Corset according to one of claims 1 to 5, **characterised in that** the upper flat pressure body (110, 210, 310, 410) is in the shape of a substantially transverse extending yoke.

7. Corset according to one of claims 1 to 6, **characterised in that** the two longitudinal supports become wider from their lower end towards the lower flat pressure body.

8. Corset according to one of claims 1 to 7, **characterised in that** the upper flat pressure body (110, 210, 310, 410) merges in each case into the longitudinal supports (120, 220, 320, 420) in the lateral outside regions.

9. Corset according to one of claims 1 to 8, **characterised in that** the upper flat pressure body (110, 210, 310) merges into an arched region (112, 212, 312) rearward and downward at the lateral end.

10. Corset according to one of claims 1 to 9, **characterised in that** the central rear pressure body (150, 250, 350) is connected to the front pressure body (110, 130; 210, 230; 310, 330).

11. Corset according to claim 9, **characterised in that** the central rear pressure body (150, 250, 350) is connected to the arched region (112, 212, 312)..

12. Corset according to one of claims 1 to 11, **characterised in that** the arched region (112, 212, 312) is configured at the level of the front armpit longitudinal line in such a manner that an expansion space is provided for the trunk volume that is redressed forwards.

13. Corset according to claim 12, **characterised in that** the arched region (112, 212, 312) includes stiffening means in the region of the expansion space.

14. Corset according to one of claims 1 to 13, **characterised in that** the closure (160, 260, 360) is provided at the side between the central rear pressure body and the front pressure bodies.

15. Corset according to one of claims 3 and 4 to 11, **characterised in that** the closure (480, 490) is provided at the front.

16. Corset according to claim 15, **characterised in that** the longitudinal supports (120, 220, 320, 420) are curved gently forwards.

17. Corset according to one of claims 1 to 16, **characterised in that** the disposition and the compressive force of the flat pressure bodies are adjustable.

18. Corset according to claims 3 to 5 and 16, **characterised in that** the divided front and/or rear flat pressure bodies (214, 234, 252; 314, 334, 352) are each connected by means of an adjusting mechanism.

19. Corset according to one of claims 1 to 18; **characterised in that** an adjusting mechanism (170, 270, 370) is provided between the front and the rear flat pressure bodies.

20. Corset according to one of claims 1 to 18, **characterised in that** the front flat pressure bodies (110, 130) are in one piece.

21. Corset according to one of claims 1 to 20, **characterised in that** the rear flat pressure body (150) is in on piece.

22. Corset according to one of claims 1 to 21, **characterised in that** the said corset is in the form of a structured framework.

23. Corset according to one of claims 1 to 21, **characterised in that** the said corset is in the form of a cast structure made from plastics materials.

24. Corset according to one of claims 1 to 21, **characterised in that** the said corset is formed by means of deep drawing with profiling made from plastics material.

## Revendications

1. Corset, en particulier pour le traitement de la scoliose, comprenant une coque plane antérieure et postérieure, un espace de croisement (140, 240, 340, 440) étant prévu pour le déplacement du volume du tronc, **caractérisé en ce que**
- une coque plane antérieure inférieure (130, 230, 330, 430) est prévue dans la zone centrale,
- une coque plane antérieure supérieure (110, 210, 310, 410) est prévue, qui comporte une extension sensiblement horizontale depuis un côté vers le milieu jusqu'à l'autre côté,
- la coque plane inférieure et la coque plane supérieure (110, 130; 210, 230; 310, 330; 410) étant reliées par au moins un mât longitudinal (120, 220, 320, 420),
- et une coque plane postérieure centrale (150, 250, 350) est prévue,
- les coques étant positionnées de telle manière que lors de l'utilisation du corset la coque antérieure inférieure (130, 230, 330, 430) appuie directement au-dessus du pubis des deux côtés sur la partie la plus basse du fascia abdominal, la coque antérieure supérieure (110, 210, 310, 410) appuie d'une largeur de trois doigts sur les côtes au-dessus de l'arc costal et la partie centrale postérieure de la coque (150, 250, 350) appuie sur le milieu de la colonne lombaire.

2. Corset selon la revendication 1, **caractérisé en ce que** la coque inférieure et la coque supérieure (110, 130; 210, 230; 310, 330; 410) sont reliées par deux mats longitudinaux passant en biais (120, 220, 320, 420), qui s'étendent depuis les zones extérieures latérales de la coque antérieure supérieure (110, 210, 310, 410) jusqu'à la coque antérieure inférieure (130, 230, 330, 430) et sont cintrées vers l'avant et un espace de croisement (140, 240, 340, 440) est prévu entre la coque inférieure et la coque supérieure (110, 130; 210, 230; 310, 330; 410) et les deux mats longitudinaux (120, 220, 320, 420).

3. Corset selon la revendication 1, **caractérisé en ce que** la coque antérieure supérieure (210, 310, 410) est partagée.

4. Corset selon la revendication 1 ou 3, **caractérisé en ce que** la coque antérieure inférieure (230, 330, 430) est partagée.

5. Corset selon l'une quelconque des revendications 1 à 4, **caractérisé en ce que** la coque postérieure centrale (250, 350) est partagée.

6. Corset selon l'une quelconque des revendications 1 à 5, **caractérisé en ce que** la coque plane supérieure (110, 210, 310, 410) a la forme d'une bride passant sensiblement obliquement.

7. Corset selon l'une quelconque des revendications 1 à 6, **caractérisé en ce que** les deux mats longitudinaux sont élargis à leur extrémité inférieure vers la coque plane inférieure.

8. Corset selon l'une quelconque des revendications 1 à 7, **caractérisé en ce que** la coque plane supérieure (110, 210, 310, 410) dépasse dans les zones extérieures latérales respectivement dans les mats longitudinaux (120, 220, 320, 420).

9. Corset selon l'une quelconque des revendications 1 à 8, **caractérisé en ce que** la coque plane supérieure (110, 210, 310, 112) dépasse à l'extrémité latérale vers l'arrière et le bas dans une zone cintrée (112, 212, 312).

10. Corset selon l'une quelconque des revendications 1 à 9, **caractérisé en ce que** la coque arrière centrale (150, 250, 350) est reliée aux coques antérieures (110, 130; 210, 230; 310, 330).

11. Corset selon la revendication 9, **caractérisé en ce que** la coque centrale antérieure (150, 250, 350) est reliée à la zone cintrée (112, 212, 312).

12. Corset selon l'une quelconque des revendications 1 à 11, **caractérisé en ce que** la zone cintrée (112, 212, 312) est formée à la hauteur de la ligne longitudinale avant du creux des aisselles de telle manière qu'un espace d'expansion est mis à disposition pour le volume du tronc redressé vers l'avant.

13. Corset selon la revendication 12, **caractérisé en ce que** la zone cintrée (112, 212, 312) comporte un moyen de renforcement dans la zone de l'espace d'expansion.

14. Corset selon l'une quelconque des revendications 1 à 13, **caractérisé en ce que** la fermeture (160, 260, 360) est prévue latéralement entre la coque postérieure centrale et les coques antérieures.

15. Corset selon l'une quelconque des revendications 3 et 4 à 11, **caractérisé en ce que** la fermeture (480, 490) est prévue sur le devant.

16. Corset selon la revendication 15, **caractérisé en ce que** les mats longitudinaux (120, 220, 320, 420) sont légèrement précambrés vers l'avant.

17. Corset selon l'une quelconque des revendications 1 à 16, **caractérisé en ce que** la disposition et la force de compression de la coque plane sont variables.

18. Corset selon les revendications 3 à 5 et 16, **caractérisé en ce que** les coques planes séparées antérieures et/ou postérieures (214, 234, 252; 314, 334, 352) sont reliées chacune par un mécanisme d'ajustage.

19. Corset selon l'une quelconque des revendications 1 à 18, **caractérisé en ce qu'**un mécanisme d'ajustage (170, 270, 370) est prévu entre la coque plane antérieure et la coque plane postérieure.

20. Corset selon l'une quelconque des revendications 1 à 18, **caractérisé en ce que** les coques planes antérieures (110, 130) sont formées de manière monobloc.

21. Corset selon l'une quelconque des revendications 1 à 20, **caractérisé en ce que** la coque plane postérieure (150) est formée de manière monobloc.

22. Corset selon l'une quelconque des revendications 1 à 21, **caractérisé en ce qu'**il est monté comme une structure à portiques.

23. Corset selon l'une quelconque des revendications 1 à 21, **caractérisé en ce qu'**il est formé comme une structure moulée en matière plastique.

24. Corset selon l'une quelconque des revendications 1 à 21, **caractérisé en ce qu'**il est formé par emboutissage avec profilage en matière plastique.
